# DEMANDE DE BREVET EUROPEEN

(11) **EP 2 561 896 A1**
(43) Date de publication de la demande: **27.02.2013**
(21) Numéro de dépôt: 12165823.1
(22) Date de dépôt: 26.04.2012
(51) Int. Cl.: A61L 15/26, A61L 15/44, A61F 13/00

(54) **Matériau composite pour dispositif médical**

(30) Priorité: 23.08.2011 US 201161526468 P
(71) Demandeur: Zodiac Automotive Division, 78373 Plaisir (FR)
(72) Inventeur: Guillo, Jean-Roger, 38300 Serezin De La Tour (FR); Roland, Sophie, 38230 Tignieu (FR); Chartron, Vincent, 69220 Belleville (FR)
(74) Mandataire: Zimmermann, Alain

(57) **Abrégé**

L'invention concerne un matériau composite, en particulier un matériau destiné à être utilisé dans un dispositif médical tel qu'un pansement, comprenant une couche comportant une mousse absorbante et une couche présentant des ouvertures et un gel à base de silicone imprégnant le support textile sans remplir les ouvertures dudit support textile. L'invention concerne également un procédé de fabrication du et dispositif médical le comprenant.

## Description

L'invention concerne un matériau composite, en particulier un matériau destiné à être utilisé dans un dispositif médical tel qu'un pansement, ainsi que son procédé de fabrication. L'invention concerne également un dispositif médical comprenant ledit matériau composite.

On connaît des dispositifs médicaux qui sont appliqués sur une zone cutanée à protéger présentant une lésion, par exemple un pansement. Ces dispositifs permettent d'isoler la zone cutanée à protéger de toute contamination extérieure par des fluides environnants tels que l'eau, par des substances chimiques telles que le savon, les graisses, ainsi que par des agents de contamination bactérienne ou micro-organismes. Ces dispositifs permettent également d'évacuer vers l'extérieur l'excédent d'eau présent à intérieur dudit dispositif et issu des sécrétions de la zone cutanée à protéger. Enfin, ils permettent de protéger la zone cutanée recouverte de toute agression mécanique extérieure.

Généralement, un tel dispositif comprend une masse absorbante qui permet d'absorber les sécrétions de la zone cutanée à protéger, tels que les exsudats d'une plaie, une couche adhésive qui permet au dispositif d'adhérer à la zone cutanée péri-lésionnelle et d'être maintenu autour de cette zone, et éventuellement une couverture formant la surface extérieure dudit dispositif.

Ainsi, la masse absorbante absorbe les liquides sécrétés par la zone cutanée.

Éventuellement, elle peut aussi contenir des principes actifs, destinés à traiter la zone cutanée. On connaît, par le document WO 87/05206, un matériau pour dispositif médical destiné être en contact avec une plaie, comprenant une structure ouverte, telle qu'un tricot, enduite d'un adhésif hydrophobe doux, qui adhère à la zone péri-lésionnelle mais pas à la plaie.

Cet adhésif doux a pour avantage de permettre que le retrait du matériau composite ne soit pas douloureux tout en favorisant la cicatrisation.

Toutefois, ce matériau a pour inconvénient d'être difficile à mettre en oeuvre en pratique. En effet, comme il ne peut absorber les exsudats de la plaie, il ne peut être employé seul et doit être utilisé conjointement avec une mousse absorbante et une bande de maintien, l'ensemble formant le dispositif médical.

Or, ces différents éléments ne peuvent être assemblés qu'au moment de la mise en place du dispositif, car ils ne présentent pas suffisamment d'adhésion entre eux pour permettre une cohésion dans la durée, ce qui nécessite un remplacement fréquent du dispositif médical.

Par ailleurs, on connaît, par le document WO 97/42985, un matériau pour dispositif médical comprenant une mousse absorbante présentant des ouvertures non traversantes, enduite d'un gel adhésif hydrophobe qui remplit partiellement les ouvertures sans les boucher.

Toutefois, ce matériau présente l'inconvénient d'être difficile à fabriquer, car la réalisation de trous non traversants dans la mousse absorbante puis son enduction partielle de manière à ne pas boucher le fond des trous sont des opérations délicates.

De plus, l'interface avec la zone cutanée est uniquement assurée par le gel adhésif hydrophobe doux qui est peu cohésif, ce qui peut conduire à laisser des résidus sur la peau lors du retrait du dispositif médical.

Le but de la présente invention est donc de fournir un matériau composite pour dispositif médical, destiné à venir en contact avec une plaie, facile à fabriquer et à intégrer dans un dispositif médical.

A cet effet, l'invention concerne un matériau composite comprenant une couche comportant une mousse absorbante et une couche comportant un support textile présentant des ouvertures et un gel à base de silicone, ledit gel de silicone imprégnant le support textile sans remplir les ouvertures dudit support textile.

Le matériau selon l'invention permet de bénéficier de l'adhésion douce du gel de silicone et du pouvoir absorbant de la mousse dans un seul ensemble unitaire présentant suffisamment de cohésion pour être stocké, manipulé, utilisé puis retiré sans que les différentes couches ne se séparent.

En effet, le matériau selon l'invention est un matériau composite, c'est-à-dire qu'il est composé de plusieurs éléments qui forment néanmoins une unité qui conserve une bonne cohésion.

Le gel de silicone imprégnant le support textile possède des propriétés adhésives qui permettent de maintenir ensemble le support textile enduit et la mousse absorbante.

De plus, le gel de silicone est un adhésif hydrophobe doux qui adhère à la peau saine mais pas à une plaie (libérant des exsudats aqueux), de sorte qu'il n'est pas nécessaire de prévoir que le gel ne soit présent qu'au niveau des zones du matériau qui ne sont pas destinées à entrer en contact avec une plaie.

Enfin, même si le gel de silicone est hydrophobe, il peut imprégner toute la surface de la structure textile, puisque celle-ci comporte des ouvertures permettant aux exsudats d'une plaie d'atteindre la mousse absorbante.

D'autre part, l'emploi d'un support textile rend l'interface entre le matériau de l'invention et la peau beaucoup plus résistante qu'un gel de silicone employé seul.

En effet, le gel de silicone est supporté par la structure textile ce qui permet de répartir les contraintes sur toute la surface enduite lors du retrait.

Enfin, le matériau composite selon l'invention est de réalisation aisée, comme indiqué plus bas.

Selon un mode de réalisation de l'invention, la mousse absorbante est réalisée dans un matériau hydrophobe et perméable aux fluides.

Selon un mode de réalisation préféré de l'invention, ladite mousse absorbante est à base de polyuréthane.

Par exemple, la mousse absorbante est à base d'un polyuréthane réticulé hydrophobe et ne présentant pas de gonflement lors de l'absorption de fluides.

Alternativement, ladite masse absorbante est à base de non tissé de fibres (par exemple à base de cellulose, de polypropylène).

La mousse absorbante utilisée présente typiquement un niveau d'absorption de 10 et 30 g absorbé par /g de mousse.

Elle présente de préférence une épaisseur comprise entre 1 et 10 mm.

Alternativement, la mousse absorbante présente une épaisseur inférieure à 1 mm, notamment lorsque les besoins en termes d'absorption sont peu importants ou que le matériau est destiné à être intégré dans un complexe comprenant une mousse absorbante supplémentaire.

La densité de la mousse est généralement de l'ordre de 100kg/ₘ³_{.}

On citera à titre d'exemples la mousse Lohman et RauscherSuperasorb X,la mousse Corpura/AMS commercialisée sous la référence MCF03, ou la mousse MS 50PW de Filtrona.

Selon un mode de réalisation de l'invention, ladite mousse contient des principes actifs thérapeutiques et/ou prophylactiques qui permettent par exemple, de traiter, d'accélérer la cicatrisation ou de prévenir l'infection de la zone cutanée sur laquelle le matériau selon l'invention sera posé.

Par exemple, on pourra choisir une mousse capable de réguler le taux d'humidité à la surface de la plaie sur laquelle le matériau composite de l'invention sera appliqué, favorisant ainsi la cicatrisation

De façon générale, on choisira la mousse absorbante en fonction de la typologie de la plaie sur laquelle le matériau composite selon l'invention devra être appliqué (plaie faiblement ou fortement exsudative).

De préférence, la structure textile utilisée dans le matériau composite est une structure tricotée.

En effet, le tricot a pour avantage de permettre une grande flexibilité, ce qui permet au matériau d'être conformable et de s'adapter à des zones cutanées non planes.

De plus, les technologies de tricotage permettent d'obtenir des types de maillages très variés, avec une gamme de taille d'ouverture très large, ce qui n'est pas possible en utilisant un gel hydrophobe perforé seul.

La structure textile comprend de préférence des filaments continus en un matériau synthétique, par exemple à base de polyester ou de polyamide.

Le gel de silicone entrant dans la composition du matériau composite selon l'invention est choisi parmi les silicones de grade médical.

Des exemples de tels silicones disponibles dans le commerce comprennent le silicone commercialisé par la société Dow Corning, sous la référence MG 7-9800 ou le gel de silicone commercialisé par la société BluestarSilicone, sous la référence HC2-2030.

La force d'adhésion du matériau composite sur une zone cutanée à protéger dépend du gel de silicone choisi ainsi que de son grammage. L'homme du métier saura adapter la quantité de gel de silicone à utiliser en fonction de sa nature, de façon à obtenir une force d'adhésion du matériau composite à la peau de l'ordre de 0,1 à 2 N/25 mm.

Le gel de silicone peut imprégner la structure textile sur toute sa surface : puisque celle-ci présente des ouvertures, la circulation des liquides sécrétées par une zone cutanée lésée ne sera pas bloquée.

En variante, le gel de silicone n'imprègne qu'une partie de la surface de la structure textile, par exemple les zones qui ne sont pas destinées à entrer en contact avec une plaie (zones péri-lésionnelles).

L'invention concerne également un dispositif médical pour protéger une zone cutanée, tel qu'un pansement, comprenant le matériau composite décrit ci-dessus la couche comportant un support textile étant destinée à être placée en regard de la zone cutanée à protéger.

Le matériau composite selon l'invention peut être directement utilisé en tant que dispositif médical sur une zone cutanée à protéger.

En variante, le dispositif médical selon l'invention comprend le matériau composite décrit plus haut et d'autres composants.

Ainsi, le dispositif médical peut être réalisé en assemblant le matériau composite selon l'invention avec une couverture extérieure, par exemple une couverture extérieure collante qui rend le dispositif étanche à l'eau et aux bactéries, permettant ainsi son port prolongé.

Selon un autre exemple, le matériau composite selon l'invention peut être intégré dans un dispositif médical comprenantune masse absorbante, par exemple une masse absorbante présentant des capacités d'absorption particulièrement élevées.

On choisira alors la mousse absorbante du matériau composite de façon à ce qu'elle soit hydrophobe mais perméable auxfluides, et de préférence non gonflante.

En effet, on connaît des dispositif médicaux du type comprenant une masse absorbante prise en sandwich entre deux couches de mousse, une des couches de mousse constituant l'interface avec la zone cutanée à protéger et l'autre constituant la couverture extérieure du dispositif.

Toutefois, il est malaisé de faire adhérer l'interface au contact de la zone cutanée de ces dispositifs.

C'est pourquoi, il est particulièrement avantageux de remplacer la mousse faisant interface avec la zone cutanée par le matériau composite selon l'invention, ce qui permet au dispositif médical d'adhérer à la zone cutanée mais pas à une plaie tout en restant perméable aux fluides.

De plus, le dispositif ainsi obtenu présente une excellente étanchéité à l'air.

Le matériau composite et la masse absorbante peuvent être assemblés sans liaison chimique, **par mouillage et accrochage mecanique du gel de silicone dans les pores de la masse absorbante** , ou au contraire par liaison chimique, par exemple **par enduction d'un primaire d'adhesion à base de siliocne et d'un silane reactif assurant la liaison chimique entre les fonctions du silicone et celle de la masse absorbante.**

Le matériau composite selon l'invention présentant une bonne résistance thermique, ce dernier et la couche de mousse faisant office de couverture extérieure peuvent être assemblés par soudure périphérique, par exemple par soudure thermique ou soudure UHF

Le dispositif ainsi obtenu présente une excellente étanchéité à l'air.

De ce fait, le matériau composite selon l'invention peut être utilisé dans des dispositifs du type de ceux décrits ci-dessus, et munis en outre d'une tétine creuse de sortie traversant la couverture extérieure dudit dispositif et permettant la succion des fluides absorbés par la masse absorbante et l'application d'une pression négative sur la plaie à l'aide d'un dispositif de mise sous vide.

Ce type de dispositif, connu sous le nom de NPW (Negative pressure wounddressing ou pansement à pression négative) est connu pour favoriser la cicatrisation, notamment dans le cas de plaies présentant une forte exsudation.

Dans un autre cas de figure, le dispositif médical peut comprendre le matériau composite décrit plus haut ainsi qu'une bande ou un filet de maintien.

L'invention concerne enfin un procédé de fabrication du matériau composite décrit ci-dessus, ledit procédé comprenant les étapes consistant à :
- enduire une structure textile présentant des ouvertures d'une solution de pré-polymère de silicone,
- déposer sur une face de ladite structure textile enduite une mousse absorbante à base de polyuréthane, et
- polymériser ladite solution de pré-polymère.

Le procédé selon l'invention a pour avantage de permettre de créer une structure complexée présentant une cohésion particulièrement bonne.

En effet, le fait d'assembler la structure textile enduite d'une solution de pré-polymère avec la mousse absorbante avant de procéder à la polymérisation permet à la solution de pré-polymère d'être partiellement absorbée en surface par la mousse absorbante.

Ainsi, lorsque l'on procède à la polymérisation, ceci conduit à la vulcanisation de la solution de pré-polymère, et génère des accroches mécaniques entre la mousse absorbante et la structure textile.

Lorsque la mousse absorbante est réalisée à base de polyuréthane, il se crée également un phénomène d'accroche chimique entre les chaînes du silicone et celles du polyuréthane.

Du fait de ces phénomènes d'accroche, l'adhésion entre la structure textile et la masse absorbante est irréversible. La structure textile et la mousse absorbante forment alors un matériau composite unitaire qui ne risque pas de se désolidariser.

Dans le cadre de l'invention, on peut également employer des traitements physiques, tel que le traitement Corona,qui permettent d'augmenter significativement l'accroche entre le gel et la masse absorbante dans la plupart des cas.

L'étape d'enduction peut être, par exemple, réalisée par enduction à la racle, au cylindre ou par foulardage.

Selon la viscosité de la solution de pré-polymère employée, celle-ci sera plus ou moins absorbée par la mousse absorbante, ce qui permet de faire varier la force de cohésion du matériau composite en fonction des applications envisagées.

La quantité de solution de pré-polymère de silicone à appliquer lors de l'étape d'enduction est fonction de plusieurs facteurs et en particulier de la viscosité du silicone.Généralement, la quantité de silicone appliquée lors de l'étape d'enduction est de 100 à 1000 g/m², en fonction des propriétés du silicone employé et de la force d'adhésion matériau composite-zone cutanée recherchée (typiquement de l'ordre de 0,1 à 2N)

Les paramètres de mise en oeuvre de cette étape d'enduction, tels que la vitesse d'enduction, sont à adapter en fonction de la capacité d'absorption de la masse absorbante ainsi que de sa nature, afin de ne pas la saturer en silicone ni de la dégrader.

En effet, la solution de pré-polymère de silicone doit imprégner la mousse absorbante uniquement en surface, pour permettre à celle-ci de conserver sa capacité à absorber les exsudats d'une plaie lorsque le matériau composite est posé sur une zone cutanée lésée.

L'enduction peut être continue, sur toute l'intégralité de la structure textile ou sur seulement une partie.

Selon un mode de réalisation de l'invention, entre l'étape d'enduction de la structure textile et l'étape de dépôt de la mousse absorbante, on procède à une étape consistant à désobstruer les ouvertures de la structure textile remplies de la solution de pré-polymère.

Ce mode de réalisation est particulièrement adapté lorsque la solution de pré-polymère employée présente une viscosité élevée.

L'étape de polymérisation est effectuée selon des procédés classiques, en particulier dans un four à air chaud, en fixant une température que la mousse absorbante puisse supporter sans se dégrader.

L'invention va maintenant être décrite plus en détail à l'aide des figures annexées dans lesquelles :
- la figure 1 est une vue en coupe d'un matériau composite selon l'invention,
- la figure 2 est une vue de dessus du matériau composite de la figure 1,
- la figure 3 est une vue en coupe d'un dispositif médical selon l'invention
- la figure 4 est une vue en coupe d'un mode de réalisation alternatif d'un dispositif médical selon l'invention, et
- la figure 5 est une vue en coupe d'une variante du dispositif médical de la figure 4.

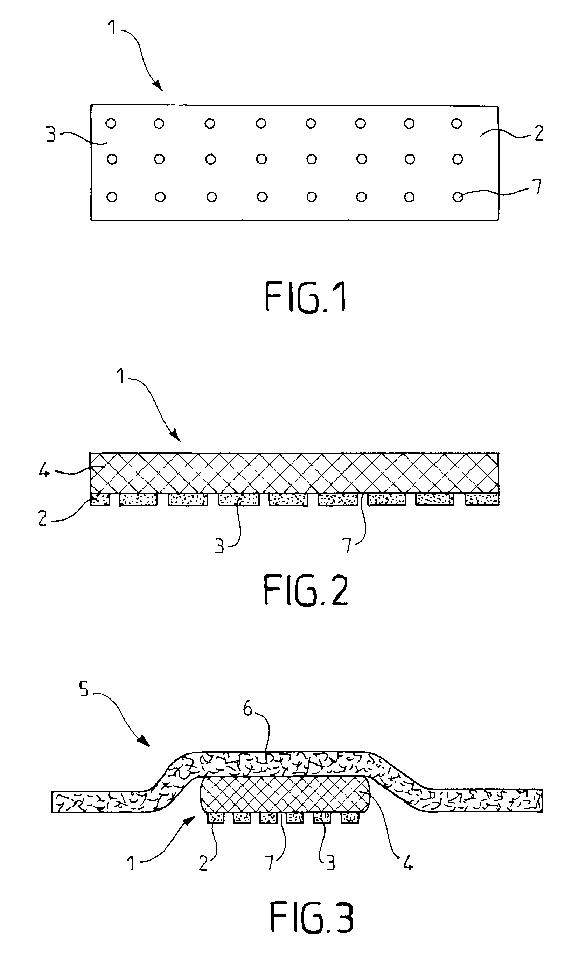

La figure 1 et la figure 2 représentent un matériau composite 1 selon l'invention.

Le matériau composite 1 est formé d'une couche textile 2 présentant des ouvertures 7, enduite d'un gel de silicone 3 et d'une mousse absorbante 4.

Ici, la couche textile 2 est une couche en un tricot de filaments de polyester continus.

Cette couche tricotée 2 est enduite sur toute sa surface d'un gel de silicone de grade médical 3 qui ne bouche pas les ouvertures 7.

Le gel de silicone permet d'assurer la cohésion avec la mousse absorbante 4, réalisée à base de polyuréthane, dont il imprègne la surface.

La figure 3 représente un dispositif médical, ici un pansement 5 comprenant le matériau composite 1 décrit aux figures 1 et 2, ainsi qu'un couverture extérieure 7, la mousse absorbante 4 étant placée en regard de la couverture extérieure 7.

Cette couverture extérieure 7 est ici en silicone.

Une fois le pansement 5 en place sur une zone cutanée à protéger, la couche tricotée 2 imprégnée de gel de silicone 3 vient en contact avec ladite zone cutanée à protéger,présentant par exemple une plaie libérant des exsudats et une zone péri-lesionnelle.

Le gel de silicone 3 étant hydrophobe, il n'adhère pas à la plaie libérant des exsudats tandis que l'adhésion entre la zone péri-lésionnelle et le pansement 5 est douce, de sorte que le retrait dudit pansement 5 n'est pas douloureux.

Du fait que le gel de silicone ne remplit pas les ouvertures 7, les exsudats pourront les traverser et être absorbés par la mousse absorbante 4.

Même une fois gorgée par les exsudats, la mousse absorbante 4 reste solidaire de la couche tricotée 2 enduite de gel de silicone 3, grâce au gel de silicone qui imprègne à la fois la couche tricotée 2 et la surface de la mousse absorbante 4.

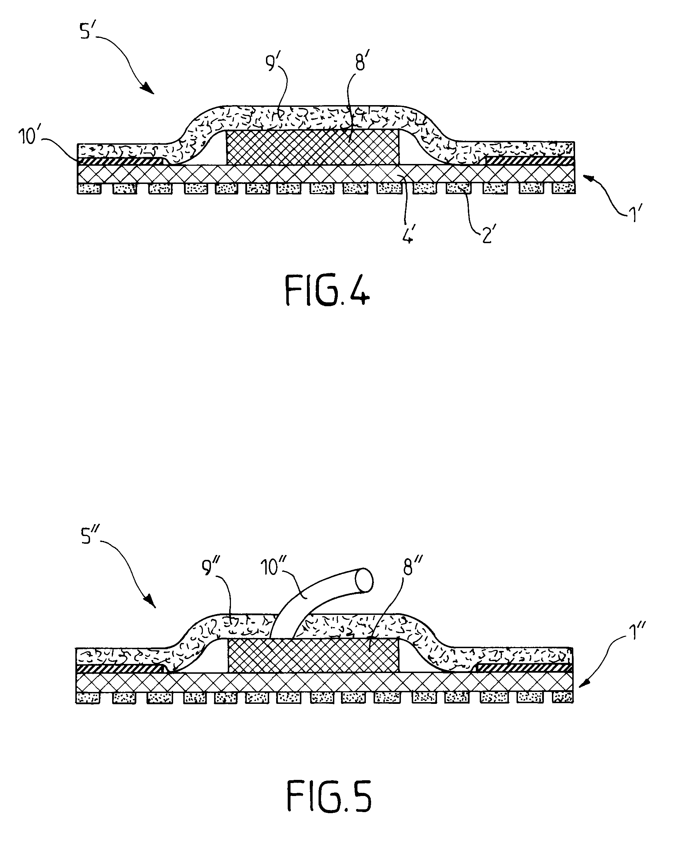

La figure 4 représente un autre mode de réalisation d'un dispositif selon l'invention, comprenant le matériau composite 1', destiné à former l'interface avec une zone cutanée à protéger, une masse absorbante 8' et une couverture extérieure 9' du dispositif.

Le matériau composite 1' comprend un tricot 3 imprégné de silicone et une mousse absorbante 4.

La couvertureextérieure 9'du dispositif 5' est réalisée en mousse absorbante et joue le rôle d'une membrane perméable à la vapeur. Elle est par exemple en polyuréthane hydrophobe.

La masse absorbante 8' présente des capacités d'absorption très élevées et est par exemple réalisée à base de polyuréthane hydrophile.

La masse absorbante 8' est prise en sandwich entre le matériau composite 1' et la mousse absorbante 9', lesquelles sont assemblées l'une à l'autre par soudure périphérique (représentée sur la figure 4 par les zones de soudure 10').

Ainsi, une fois le dispositif médical 5' disposé sur une zone cutanée à protéger, les fluides sécrétés traversent le matériau composite l'et peuvent être absorbés en grande quantité par la masse absorbante 8'.

La figure 5 représente une variante 5" du dispositif médical 5', comprenant, outre un matériau composite 1", une masse absorbante 8" et une couverture extérieure 9", un tétine creuse de succion et mise sous vide 10".

Cette tétine est disposée sur la masse absorbante 8" et traverse la couverture extérieure 9".

Ce type de dispositif est particulièrement avantageux pour traiter des zones cutanées présentant des plaies particulièrement exsudatives, la mise en place de la tétine permettant, à l'aide d'un dispositif de mise sous vide, de créer une pression négative sur la plaie de sorte que les liquides sont évacuées très efficacement.

## Revendications

1. Matériau composite comprenant une couche comportant une mousse absorbante et une couche comportant un support textile présentant des ouvertures et un gel à base de silicone, ledit gel de silicone imprégnant le support textile sans remplir les ouvertures dudit support textile.

2. Matériau composite selon la revendication 1, **caractérisé en ce que** ladite mousse absorbante est à base de polyuréthane.

3. Matériau composite selon l'une quelconque des revendications 1 et 2, **caractérisé en ce que** ladite structure textile est une structure tricotée.

4. Matériau composite selon l'une quelconque des revendications 1 à 3, **caractérisé en ce que** ledit support textile comprend des filaments continusen un matériau synthétique.

5. Matériau composite selon l'une quelconque des revendications 1 à 4, **caractérisé en ce que** la mousse absorbante à base de polyuréthane présente une épaisseur comprise entre 1 et 10 mm.

6. Matériau composite selon l'une quelconque des revendications 1 à 4, **caractérisé en ce que** la mousse absorbante à base de polyuréthane contient des principes actifs thérapeutiques et/ou prophylactiques.

7. Dispositif médical pour protéger une zone cutanée, tel qu'un pansement, comprenant le matériau composite selon l'une quelconque des revendications 1 à 6, la couche comportant un support textile étant destinée à être placée en regard de la zone cutanée à protéger.

8. Procédé de fabrication d'un matériau composite selon l'une quelconque des revendications 1 à 6, comprenant les étapes consistant à :
- enduire une structure textile présentant des ouvertures d'une solution de pré-polymère de silicone,
- déposer sur une face de ladite structure textile enduite une mousse absorbante à base de polyuréthane, et
- polymériser ladite solution de pré-polymère.

9. Procédé de fabrication selon la revendication 8, **caractérisé en ce que** l'étape d'enduction est réalisée par enduction à la racle, au cylindre ou par foulardage.

10. Procédé selon l'une quelconque des revendications 8 et 9, **caractérisé en ce que** ledit procédé comprend en outre, entre l'étape d'enduction de la structure textile et l'étape de dépôt de la mousse absorbante, une étape consistant à désobstruer les ouvertures de la structure textile remplies de la solution de pré-polymère.
